# EUROPÄISCHE PATENTANMELDUNG

(11) **EP 3 608 912 A1**
(43) Veröffentlichungstag der Anmeldung: **12.02.2020**
(21) Anmeldenummer: 18187434.8
(22) Anmeldetag: 06.08.2018
(51) Int. Cl.: G16B 20/20, G16B 40/00, G16H 50/80

(54) **ERMITTELN EINER SUBSTANZKLASSE EINES NOSOKOMIALEN KEIMS**

(71) Anmelder: Siemens Healthcare GmbH, 91052 Erlangen (DE)
(72) Erfinder: Dietrich, Carsten, 90408 Nürnberg (DE); Siebert, Matthias, 91080 Marloffstein (DE)

(57) **Zusammenfassung**

Die Erfindung betrifft ein Verfahren für ein Ermitteln einer Substanzklasse eines nosokomialen Keims in einer biologischen Testsubstanz eines Patienten, ein zugehöriges medizinisches System und ein zugehöriges Computerprogrammprodukt.

Das erfindungsgemäße Verfahren für ein Ermitteln einer Substanzklasse eines nosokomialen Keims in einer biologischen Testsubstanz eines Patienten weist folgende Schritte auf:
- Bereitstellen eines Datenbanksystems, welches eine Menge an Substanzklassen aufweist, wobei jeder Substanzklasse zumindest ein nosokomialer Keim zugeordnet ist und zumindest eine Substanzklasse aus der Menge an Substanzklassen Protokolldaten über ein Vorkommen des zugeordneten zumindest einen nosokomialen Keims aufweist,
- Bereitstellen der biologischen Testsubstanz des Patienten zusammen mit Ursprungsdaten, welche einen Ursprung der biologischen Testsubstanz beschreiben,
- Festlegen von Startdaten durch ein Gewichten der Protokolldaten aus der Menge an Substanzklassen in Abhängigkeit von den Ursprungsdaten,
- Ermitteln einer Schätzfunktion mittels einer Analyseeinheit, wobei die Startdaten und die biologische Testsubstanz als Eingangsparameter in die Analyseeinheit eingehen und die Schätzfunktion an einem Ausgang der Analyseeinheit bereitgestellt wird, und
- Ermitteln der Substanzklasse des nosokomialen Keims in der biologischen Testsubstanz des Patienten gemäß der Schätzfunktion.

## Beschreibung

Die Erfindung betrifft ein Verfahren für ein Ermitteln einer Substanzklasse eines nosokomialen Keims in einer biologischen Testsubstanz eines Patienten, ein zugehöriges medizinisches System und ein zugehöriges Computerprogrammprodukt.

Ein für eine Infektion eines Patienten ursächlicher Keim wird üblicherweise in einer herkömmlichen Analyseeinheit, insbesondere in einer herkömmlichen biologischen Testsubstanzauswerteeinheit, mittels eines kultivierungsabhängigen Verfahrens ermittelt. Alternativ oder zusätzlich kann der Keim kultivierungsunabhängig in der herkömmlichen Analyseeinheit, insbesondere in der biologischen Testsubstanzauswerteeinheit, beispielsweise mittels einer Polymerase-Kettenreaktion mit pathogen-spezifischen Oligonukleotiden, ermittelt werden. Grundsätzlich ist es denkbar, dass der Keim mittels einer Sequenzierung von einem Desoxyribonukleinsäure-Molekül und/oder von einem Ribonukleinsäure-Molekül ermittelt wird. Eine Analyse der bei der Sequenzierung ermittelten Sequenzierungsdaten hängt üblicherweise von proprietären und/oder öffentlichen Referenzdaten ab, welche typischerweise nicht spezifisch für Ort und Zeit und gleichzeitig statisch ausgebildet sind. Die Referenzdaten sind beispielsweise Teil einer GEAR- oder CARD-Datenbank.

Keime mit multiplen Resistenzen sind insbesondere für ca. 6% der in einem Krankenhaus erworbenen, sprich nosokomialen, Infektionen verantwortlich. In anderen Worten können die Keime mit multiplen Resistenzen grundsätzlich in dem Krankenhaus auf einen Patienten übertragen werden. Aufgrund von mit dem Resistenzspektrum verbundenen Einschränkungen bei einer Behandlung des Patienten, ist üblicherweise das Ermitteln der Substanzklasse des nosokomialen Keims zeitkritisch.

Der Erfindung liegt die Aufgabe zu Grunde, ein Verfahren für ein Ermitteln einer Substanzklasse eines nosokomialen Keims in einer biologischen Testsubstanz eines Patienten, ein zugehöriges medizinisches System und ein zugehöriges Computerprogrammprodukt anzugeben, bei welchen eine Zeit für das Ermitteln der Substanzklasse des nosokomialen Keims verkürzt ist.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Ermitteln einer Substanzklasse eines nosokomialen Keims in einer biologischen Testsubstanz eines Patienten weist folgende Schritte auf:
- Bereitstellen eines Datenbanksystems, welches eine Menge an Substanzklassen aufweist, wobei jeder Substanzklasse zumindest ein nosokomialer Keim zugeordnet ist und zumindest eine Substanzklasse aus der Menge an Substanzklassen Protokolldaten über ein Vorkommen des zugeordneten zumindest einen nosokomialen Keims aufweist,
- Bereitstellen der biologischen Testsubstanz des Patienten zusammen mit Ursprungsdaten, welche einen Ursprung der biologischen Testsubstanz beschreiben,
- Festlegen von Startdaten durch ein Gewichten der Protokolldaten aus der Menge an Substanzklassen in Abhängigkeit von den Ursprungsdaten,
- Ermitteln einer Schätzfunktion mittels einer Analyseeinheit, wobei die Startdaten und die biologische Testsubstanz als Eingangsparameter in die Analyseeinheit eingehen und die Schätzfunktion an einem Ausgang der Analyseeinheit bereitgestellt wird, und
- Ermitteln der Substanzklasse des nosokomialen Keims in der biologischen Testsubstanz des Patienten gemäß der Schätzfunktion.

Das erfindungsgemäße Verfahren bietet insbesondere folgende Vorteile:
Die Protokolldaten über das Vorkommen des zumindest einen nosokomialen Keims und die Ursprungsdaten der biologischen Testsubstanz sind vorteilhaft, weil dadurch das Ermitteln der Substanzklasse schneller erfolgen kann und/oder eine Historie des zumindest einen nosokomialen Keims berücksichtigt wird.
Das Festlegen der Startdaten in Abhängigkeit von den Ursprungsdaten ermöglicht vorteilhafterweise ein Berücksichtigen von solchen Rahmenbedingungen, in welchen die biologische Testsubstanz bereitgestellt wird, wodurch vorteilhafterweise ein kontextbezogenes Ermitteln der Substanzklasse des nosokomialen Keims erfolgt. Dies ermöglicht vorteilhafterweise ein besseres Ergebnis, weil üblicherweise ein Zusammenhang zwischen den Protokolldaten und den Ursprungsdaten besteht.

Das Ermitteln der Schätzfunktion weist insbesondere ein probabilistisches Verfahren auf und ermöglicht daher eine Berücksichtigung einer stochastischen Natur von biologischen Prozessen, welche den nosokomialen Keim beschreiben können. Die Schätzfunktion ermöglicht weiterhin, insbesondere in Abhängigkeit von einer Sensitivität und Spezifizität, einen Zeitpunkt für das Ermitteln der Substanzklasse vorzugeben. Vorteilhafterweise kann in Abhängigkeit von einem Gesundheitszustand des Patienten der Zeitpunkt für das Ermitteln der Substanzklasse vorgegeben werden, beispielsweise indem eine Anforderung an die Sensitivität und/oder eine Anforderung an die Spezifität relativ zum Gesundheitszustand angepasst wird. Typischerweise kann ein Arzt desto früher den Patienten gemäß der ermittelten Substanzklasse behandeln, je früher der Zeitpunkt für das Ermitteln der Substanzklasse vorgegeben wird.

Besonders vorteilhafterweise ermöglicht das erfindungsgemäße Verfahren ein patientenindividuelles Ermitteln der Substanzklasse des nosokomialen Keims in der biologischen Testsubstanz unter Berücksichtigung des Ursprungs der biologischen Testsubstanz. Vorzugsweise erfolgt das Ermitteln der Substanzklasse gemäß einem kultivierungsunabhängigen Verfahren, wodurch im Vergleich zu dem kultivierungsabhängigen Verfahren eine Ermittlungszeit für das Ermitteln der Substanzklasse von mehreren Tagen auf wenige Stunden reduziert sein kann.

Wenn die biologische Testsubstanz bereitgestellt wird und/oder die Substanzklasse des nosokomialen Keims in der biologischen Testsubstanz ermittelt wird, können vorteilhafterweise weitere Patienten vor dem nosokomialen Keim bewahrt werden, beispielsweise indem eine bevorzugte Behandlungsmethode und/oder eine unvorteilhafte Behandlungsmethode in Abhängigkeit von der ermittelten Substanzklasse bereitgestellt wird. Vorteilhafterweise kann der Patient besser und/oder schneller behandelt werden, wodurch typischerweise eine Aufenthaltsdauer, beispielsweise in einem Krankenhaus gemäß dem Ursprung der biologischen Testsubstanz, verringert wird und wobei eine Wahrscheinlichkeit verringert wird, dass der Patient eine weitere Infektion erhält.

Der nosokomiale Keim ist typischerweise ein Krankenhauskeim, welcher üblicherweise bei einem Aufenthalt und/oder bei einer Behandlung des Patienten in einem Krankenhaus auf den Patienten übertragen wird. Der nosokomiale Keim kann insbesondere ein Escherichia coli, Enterococcus faecalis, Enterococcus faecium, Kebsiella pneumoniae, Pseudomonas aeruginosa und/oder Staphylococcus aureus sein. Weitere Arten und/oder Gattungen an nosokomialen Keimen sind denkbar. Typischerweise erfolgt aufgrund des Übertragens des nosokomialen Keims eine Infektion des Patienten, welche einen Gesundheitszustand des Patienten wesentlich beinträchtigen kann. Die Infektion des Patienten kann in diesem Fall nosokomiale Infektion genannt werden. Die nosokomiale Infektion beschreibt üblicherweise ausschließlich Infektionen innerhalb des Krankenhauses. Das Datenbanksystem kann grundsätzlich auch derartige Keime aufweisen, welche außerhalb des Krankenhauses auf den Patienten übertragen worden sind. Beispielsweise weisen die Protokolldaten des ermittelten nosokomialen Keims den Ursprung außerhalb des Krankenhauses auf, wenn der außerhalb des Krankenhauses auf den Patienten übertragene Keim grundsätzlich auch innerhalb des Krankenhauses vorkommen und/oder auf den Patienten übertragen werden kann, wodurch dieser Keim ein nosokomialer Keim sein kann.

Die biologische Testsubstanz weist insbesondere ein Probenmaterial auf. Die biologische Testsubstanz ist insbesondere eine klinische Testsubstanz. Die biologische Testsubstanz kann beispielsweise eine Körperflüssigkeit und/oder eine Gewebeprobe des Patienten aufweisen. Die biologische Testsubstanz weist typischerweise ein Blut, einen Speichel und/oder einen Stuhl des Patienten auf. Die biologische Testsubstanz wird üblicherweise nach dem Ermitteln der Substanzklasse verworfen.

Die Substanzklasse weist insbesondere einen Datensatz an Informationen auf, welche üblicherweise im Gegensatz zu der biologischen Testsubstanz digital im Datenbanksystem abgespeichert werden können. In anderen Worten enthält das Datenbanksystem lediglich digitale und/oder digitalisierte Informationen und/oder typischerweise nicht die biologische Testsubstanz. Das Datenbanksystem kann insbesondere die Menge an Substanzklassen und/oder die Protokolldaten beispielsweise in einer Speichereinheit des Datenbanksystems abspeichern und/oder von der Speichereinheit abrufen. Üblicherweise kann das Datenbanksystem lediglich Informationen über die biologische Testsubstanz aufweisen. Die Substanzklasse kann gemäß einer objektorientierten Programmierung ausgebildet sein.

Die Substanzklasse kann mehrere Kategorien aufweisen, in welche der Datensatz an Informationen unterteilt sein kann. Eine erste Kategorie der Substanzklasse kann ein Erkennungszeichen aufweisen, wobei das Erkennungszeichen vorzugsweise eindeutig den zumindest einen zugeordneten nosokomialen Keim identifiziert. Das Erkennungszeichen kann eine Basen- und/oder eine Aminosäurefolge und/oder eine Gen- und/oder Proteinzusammensetzung und/oder eine mikrobiologische Merkmalsliste aufweisen. Das Erkennungszeichen kann eine Beschreibung des zumindest einen zugeordneten nosokomialen Keims aufweisen. Grundsätzlich ist es denkbar, dass in Abhängigkeit einer Spezifität des Erkennungszeichens der Substanzklasse lediglich ein nosokomialer Keim oder mehrere nosokomiale Keime jeder Substanzklasse zugeordnet sind. Grundsätzlich ist es denkbar, dass in Abhängigkeit von der Spezifität des Erkennungszeichens die Substanzklasse eine Gattung und/oder eine Art des nosokomialen Keims beschreibt.

Eine zweite Kategorie der Substanzklasse kann ein Resistenzspektrum aufweisen, welches bevorzugte und/oder unvorteilhafte, insbesondere medikamentöse, Behandlungsmethoden beschreibt. Beispielsweise kann nach dem Ermitteln der Substanzklasse aus dem Datenbanksystem das der ermittelten Substanzklasse zugeordnete Resistenzspektrum auf einer Anzeigeeinheit bereitgestellt werden. Üblicherweise wird ein Anteil an unvorteilhaften Behandlungsmethoden auf Kosten eines Anteils der bevorzugten Behandlungsmethoden immer größer. Das Resistenzspektrum kann ein Protein, ein Gen, eine Mutation und/oder ein Methylierungsmuster umfassen. Wenn ein Keim ein Resistenzspektrum mit zumindest einer Resistenz aufweist, kann der Keim mit dem Resistenzspektrum der nosokomiale Keim sein.

Eine dritte Kategorie der Substanzklasse kann einen Stammbaum aufweisen, welcher beispielsweise die Menge der Substanzklassen in Abhängigkeit von einem Verwandtschaftsgrad zwischen den Substanzklassen beschreibt. Der Verwandtschaftsgrad ist insbesondere im Hinblick auf das Resistenzspektrum vorteilhaft, weil üblicherweise Selektionsdruck und/oder genetische Plastizität eine Weiterentwicklung des Resistenzspektrums des zugeordneten nosokomialen Keims bewirken kann.

Eine vierte Kategorie der Substanzklasse kann die Protokolldaten aufweisen. Die Protokolldaten, insbesondere patientenspezifische Daten der Protokolldaten, können beispielsweise anonymisiert und/oder pseudonymisiert werden. Die Protokolldaten können insbesondere eine Biozönose des zumindest einen nosokomialen Keims aufweisen. Das Vorkommen des zugeordneten zumindest einen nosokomialen Keims beschreibt insbesondere eine Historie des zugeordneten zumindest einen nosokomialen Keims. Grundsätzlich ist es denkbar, dass die Menge an Substanzklassen, insbesondere jeder zugeordnete nosokomiale Keim, mehrere Male vorkommt. Die Protokolldaten beschreiben insbesondere Rahmenbedingungen über das Vorkommen des zugeordneten zumindest einen nosokomialen Keims.

Die Menge an Substanzklassen und insbesondere die Protokolldaten bilden typischerweise Referenzdaten, welche ein bisher bekanntes gesamtes Wissen über die nosokomialen Keime aufweisen können. Die Referenzdaten können dem Datensatz an Informationen entsprechen. Grundsätzlich kann das Datenbanksystem mehrere Datensätze an Informationen für mehrere nosokomiale Keime aufweisen.

Das Datenbanksystem kann eine lokale Datenbank und/oder eine multizentrische Datenbank und/oder eine Cloud-basierte Datenbank aufweisen. Das Datenbanksystem weist insbesondere einen Verbund der lokalen Datenbank und/oder der multizentrischen Datenbank auf. Die multizentrische Datenbank ist üblicherweise eine über mehrere Krankenhäuser verteilte Datenbank. Die mehreren Krankenhäuser können geographisch, insbesondere lokal oder regional oder national, verteilt sein. Jedes Krankenhaus kann beispielsweise eine lokale Datenbank aufweisen, wobei die lokalen Datenbanken der mehreren Krankenhäuser untereinander beispielsweise über ein Internet verbunden sind und/oder zusammen die multizentrische Datenbank bilden. Die multizentrische Datenbank ist insbesondere für ein Synchronisieren der Menge an Substanzklassen, insbesondere zusammen mit den Protokolldaten, von lokaler Datenbank zu lokaler Datenbank ausgebildet. In anderen Worten kann vorzugsweise die Menge an Substanzklassen über die mehrere Krankenhäuser, insbesondere die lokalen Datenbanken der mehreren Krankenhäuser, hinweg ausgetauscht und/oder aktualisiert werden. Jede lokale Datenbank und/oder jede multizentrische Datenbank weist üblicherweise eine Speichereinheit auf, in welche der Datensatz an Informationen, insbesondere die Menge der Substanzklassen, vorzugsweise zusammen mit den Protokolldaten, übertragen und/oder abgespeichert werden. Das Datenbanksystem kann lediglich die lokale Datenbank, beispielsweise aus an einem Ort der lokalen Datenbank anzuwendenden Datenschutzgründen, aufweisen. Das Bereitstellen des Datenbanksystems kann ein Anonymisieren der Protokolldaten umfassen. Das Bereitstellen des Datenbanksystems kann ein Verbinden, beispielsweise über das Internet und/oder ein Netzwerk, der lokalen Datenbank und/oder der multizentrischen Datenbank, insbesondere der Speichereinheit, mit der Analyseeinheit umfassen. Das Bereitstellen des Datenbanksystems kann ein Bereitstellen eines Zugriffs auf die Menge der Substanzklassen, insbesondere zusammen mit den Protokolldaten, für einen Nutzer und/oder die Analyseeinheit umfassen. Das Datenbanksystem kann beispielsweise von einem Hersteller des Datenbanksystems und/oder der Speichereinheit und/oder von dem Krankenhaus bereitgestellt werden. Das Datenbanksystem wird insbesondere in Abhängigkeit von dem Ursprung der biologischen Testsubstanz und/oder von dem den Patienten betreuenden Krankenhaus bereitgestellt.

Das Bereitstellen der biologischen Testsubstanz und/oder der Ursprungsdaten kann beispielsweise durch den Nutzer und/oder automatisch erfolgen. Beispielsweise kann der Nutzer die biologische Testsubstanz, insbesondere das Probenmaterial, von dem Patienten erfassen, beispielsweise durch eine Blutentnahme, eine Speichelprobe und/oder eine Gewebeentnahme. Das Bereitstellen der biologischen Testsubstanz und/oder der Ursprungsdaten kann das Erfassen, ein Zwischenlagern und/oder ein Aufbereiten der biologischen Testsubstanz und/oder der Ursprungsdaten umfassen. Die Ursprungsdaten können vorzugsweise automatisch bei dem Erfassen der biologischen Testsubstanz erfasst und/oder digitalisiert in dem Datenbanksystem gespeichert werden. Die Ursprungsdaten beschreiben insbesondere Rahmenbedingungen über den Ursprung der biologischen Testsubstanz.

Die Startdaten weisen üblicherweise Prior-Daten und/oder A-priori-Wissen auf. Die Startdaten können insbesondere eine A-priori-Wahrscheinlichkeit und/oder eine A-priori-Wahrscheinlichkeitsverteilung aufweisen. Die Startdaten können Trainingsdaten für die Analyseeinheit aufweisen. Die Startdaten bilden insbesondere eine Statistik in Abhängigkeit von den Protokolldaten und den Ursprungsdaten ab. Die Startdaten weisen insbesondere diejenigen Informationen auf, welche bis zu einem zeitlichen Ursprung der biologischen Testsubstanz vorliegen. Die Startdaten können beispielsweise in dem Datenbanksystem und/oder in der Analyseeinheit und/oder in einer Recheneinheit festgelegt werden. Die Startdaten können vorzugsweise zwischen dem Datenbanksystem, der Analyseeinheit und/oder der Recheneinheit ausgetauscht werden. Die Recheneinheit kann Bestandteil des Datenbanksystems und/oder der Analyseeinheit sein.

Das Gewichten der Protokolldaten kann ein Klassifizieren der Protokolldaten umfassen, wobei das Klassifizieren beispielsweise zwischen Protokolldaten mit inhaltlicher Nähe zu den Ursprungsdaten und Protokolldaten ohne inhaltliche Nähe zu den Ursprungsdaten unterscheidet. Das Klassifizieren kann durch ein Bestimmen der Gewichte für das Gewichten abgebildet werden. Die inhaltliche Nähe kann beispielsweise durch den Nutzer und/oder automatisch mittels eines Schwellwerts und/oder automatisch mittels einer weiteren Wahrscheinlichkeitsverteilung festgelegt werden. Das Gewichten der Protokolldaten kann in Abhängigkeit von einer Menge an Protokolldaten und/oder von einer Menge an Ursprungsdaten skalieren. Das Gewichten kann ein Addieren, Subtrahieren, Multiplizieren und/oder ein Potenzieren umfassen. Das Festlegen der Startdaten kann ein Abspeichern der Gewichte und/oder ein Abrufen der Gewichte in dem Datenbanksystem umfassen. Vorzugsweise werden die Gewichte in Abhängigkeit von den Ursprungsdaten bestimmt. Beispielsweise wird ein Abstandsmaß zwischen den Protokolldaten und den Ursprungsdaten festgelegt, welches das Bestimmen der Gewichte beeinflusst. Das Abstandsmaß kann einen Schwellwert umfassen.

Die Schätzfunktion kann insbesondere eine A-posteriori-Wahrscheinlichkeit und/oder eine A-posteriori-Wahrscheinlichkeitsverteilung aufweisen. Üblicherweise verändert sich die Schätzfunktion beim Ermitteln der Schätzfunktion. Die Schätzfunktion kann eine relative Häufigkeit, beispielsweise des Erkennungszeichens, aufweisen.

Die Analyseeinheit kann insbesondere die Startdaten und die biologische Testsubstanz auswerten. Die Analyseeinheit ist vorzugsweise eine kultivierungsunabhängige Analyseeinheit. Das Ermitteln der Schätzfunktion kann insbesondere in der Recheneinheit der Analyseeinheit erfolgen, wobei die Recheneinheit auf vorläufige Ergebnisse der biologischen Testsubstanz zugreifen kann. Beim Ermitteln der Schätzfunktion kann grundsätzlich unter Verwendung der A-priori-Wahrscheinlichkeitsverteilung und der biologischen Testsubstanz die A-posteriori-Wahrscheinlichkeitsverteilung ermittelt werden. Das Ermitteln der Substanzklasse, insbesondere das Ermitteln der Schätzfunktion mittels der Analyseeinheit, kann ein Vergleichen der Substanzklasse, insbesondere des Erkennungszeichens, mit der biologischen Testsubstanz umfassen. Das Vergleichen kann ein Zählen der Substanzklasse, insbesondere des Erkennungszeichens, umfassen, wodurch die relative Häufigkeit und/oder die A-posteriori-Wahrscheinlichkeitsverteilung bestimmt werden. Das Eingehen der Startdaten und der biologischen Testsubstanz als Eingangsparameter in die Analyseeinheit beschreibt insbesondere, dass die Analyseeinheit die Startdaten und die biologische Testsubstanz für das Ermitteln der Schätzfunktion verwendet.

Das Ermitteln der Substanzklasse gemäß der Schätzfunktion kann, insbesondere durch den Nutzer und/oder automatisch, mittels der Analyseeinheit erfolgen. Grundsätzlich ist es denkbar, dass ein Kriterium für das Ermitteln der Substanzklasse festgelegt wird, wobei in Abhängigkeit von dem Kriterium das Ermitteln der Substanzklasse durchgeführt, insbesondere das Ermitteln der Schätzfunktion abgeschlossen, wird. Das Ermitteln der Substanzklasse kann das Festlegen des Kriteriums umfassen. Gemäß dem Kriterium kann vorzugsweise festgelegt werden, ob die vorläufigen Ergebnisse der Schätzfunktion dafür genügen, die Substanzklasse zu ermitteln. Das Kriterium kann einen Zeitpunkt für das Ermitteln der Substanzklasse, eine Sensitivität, eine Spezifizität, eine Korrelation zwischen den Protokolldaten und den Ursprungsdaten und/oder einen Schwellwert für die A-posteriori-Wahrscheinlichkeitsverteilung, welcher beispielsweise größer 90%, insbesondere bei 99%, festgelegt wird, umfassen. Das Ermitteln der Substanzklasse umfasst insbesondere ein Bereitstellen der ermittelten Substanzklasse auf einer Anzeigeeinheit und/oder ein Abspeichern in dem Datenbanksystem und/oder ein Abspeichern in einer weiteren Datenbank außerhalb des Datenbanksystems und/oder eine Weitergabe an eine Weiterverarbeitungseinheit.

Grundsätzlich ist es denkbar, dass beim Ermitteln der Substanzklasse insbesondere das Resistenzspektrum des nosokomialen Keims ermittelt und/oder aus dem Datenbanksystem bereitgestellt wird. Alternativ oder zusätzlich ist es denkbar, dass das der ermittelten Substanzklasse zugeordnete Resistenzspektrum nach dem Ermitteln der Substanzklasse, beispielsweise dem Nutzer auf der Anzeigeeinheit, bereitgestellt wird. Die ermittelte Substanzklasse und das zugeordnete Resistenzspektrum können vorzugsweise gemeinsam auf der Anzeigeeinheit bereitgestellt werden. Vorzugsweise kann durch den Nutzer und/oder automatisch eine Behandlungsmethode für den Patienten in Abhängigkeit von dem Resistenzspektrum festgelegt und/oder beispielsweise dem Nutzer vorgeschlagen werden. Typischerweise ist das Resistenzspektrum ein Ausschlusskriterium bei dem Festlegen der Behandlungsmethode. Vorzugsweise ermöglicht das Ermitteln des Resistenzspektrums eine optimierte klinische Entscheidungsunterstützung durch die Abhängigkeit von den Ursprungsdaten und den Protokolldaten.

Eine Ausführungsform sieht vor, dass die Analyseeinheit eine biologische Testsubstanzauswerteeinheit und eine Datenverarbeitungseinheit aufweist und wobei für das Ermitteln der Schätzfunktion die biologische Testsubstanz als Eingangsparameter in die biologische Testsubstanzauswerteeinheit eingehen und die Startdaten als Eingangsparameter in die Datenverarbeitungseinheit eingehen. Vorteilhafterweise kann die Analyseeinheit die Schätzfunktion unter Verwendung der Datenverarbeitungseinheit schnell an dem Ausgang der Analyseeinheit bereitstellen.

Die Datenverarbeitungseinheit kann der Recheneinheit der Analyseeinheit entsprechen. Wenn die Analyseeinheit, insbesondere die Datenverarbeitungseinheit, und das Datenbanksystem in ein medizinisches System integriert sind, kann die Datenverarbeitungseinheit der Recheneinheit des medizinischen System und/oder der Recheneinheit des Datenbanksystems entsprechen. Die Analyseeinheit kann ein Gehäuse aufweisen, in welches die Datenverarbeitungseinheit und die biologische Testsubstanzauswerteeinheit integriert sind. Die Testsubstanzauswerteeinheit kann zu einer DNA-Sequenzierung und/oder einer RNA-Sequenzierung und/oder einer Polymerase-Kettenreaktion und/oder einer Massenspektrometrie und/oder einer Kernspinresonanzspektroskopie ausgebildet sein. Die Datenverarbeitungseinheit ist insbesondere mit dem Datenbanksystem und/oder der Recheneinheit und/oder der Anzeigeeinheit für ein Austauschen der Startdaten, der Ursprungsdaten, der Protokolldaten, insbesondere des Resistenzspektrums, und/oder der ermittelten Substanzklasse ausgebildet. Die biologische Testsubstanzauswerteeinheit kann eine Aufnahmevorrichtung für ein Aufnehmen der biologischen Testsubstanz aufweisen und/oder ist insbesondere eine kultivierungsunabhängige Testsubstanzauswerteeinheit.

Eine Ausführungsform sieht vor, dass für das Ermitteln der Schätzfunktion vorläufige Ergebnisse der Schätzfunktion zwischen der biologischen Testsubstanzauswerteeinheit und der Datenverarbeitungseinheit ausgetauscht werden. Die Ausführungsform bietet insbesondere einen Vorteil, dass zwischen der biologischen Testsubstanzauswerteeinheit und der Datenverarbeitungseinheit eine bidirektionale Kommunikationseinrichtung besteht. Beispielsweise weisen die biologische Testsubstanzauswerteeinheit und die Datenverarbeitungseinheit eine Kommunikationseinrichtung auf, welche für die bidirektionale Kommunikation ausgebildet ist. Besonders vorteilhafterweise ist die bidirektionale Kommunikationseinrichtung für eine Echtzeitkommunikation ausgebildet. Die Analyseeinheit ist vorzugsweise echtzeitfähig.

Die vorläufigen Ergebnisse der Schätzfunktion basieren vorzugsweise auf den vorläufigen Ergebnissen der biologischen Testsubstanz. Vorzugsweise entsprechen die vorläufigen Ergebnisse der Schätzfunktion einer digitalisierten Version der vorläufigen Ergebnisse der biologischen Testsubstanz. Die vorläufigen Ergebnisse der Schätzfunktion können beispielsweise das Erkennungszeichen und/oder die Substanzklasse des ermittelten nosokomialen Keims aufweisen. Die vorläufigen Ergebnisse der Schätzfunktion und/oder der biologischen Testsubstanz können vorteilhafterweise in Echtzeit zwischen der biologischen Testsubstanzauswerteeinheit und der Datenverarbeitungseinheit ausgetauscht werden. Beispielsweise kann die biologische Testsubstanzauswerteeinheit die vorläufigen Ergebnisse der Schätzfunktion und/oder die Startdaten für das Ermitteln von vorläufigen Ergebnissen der biologischen Testsubstanz verwenden.

Eine Ausführungsform sieht vor, dass für das Ermitteln der Schätzfunktion die Datenverarbeitungseinheit ein künstliches neuronales Netz aufweist. Das künstliche neuronale Netz ist insofern vorteilhaft, weil vorzugsweise das künstliche neuronale Netz an das Datenbanksystem, insbesondere an die Protokolldaten, und/oder die Ursprungsdaten angepasst werden kann. Besonders vorteilhafterweise ist das künstliche neuronale Netz ein rekurrentes künstliches neuronales Netz. Üblicherweise bietet das künstliche neuronale Netz einen Vorteil, dass Ergebnisse, insbesondere das Ermitteln der Substanzklasse, desto besser werden, je länger das künstliche neuronale Netz im Einsatz ist.

Das künstliche neuronale Netz weist üblicherweise eine Eingangsschicht und eine Ausgangsschicht auf, welche miteinander über gewichtete Verbindungen verbunden sind. Grundsätzlich können zwischen der Eingangsschicht und der Ausgangsschicht mehrere Zwischenschichten mit weiteren gewichteten Verbindungen angeordnet sein. Die Startdaten können grundsätzlich an der Eingangsschicht und/oder an der Ausgangsschicht des künstlichen neuronalen Netzes vorliegen. Die Startdaten können zwischen der Eingangsschicht und der Ausgangsschicht, beispielsweise für ein Training des künstlichen neuronalen Netzes aufgeteilt werden. Typischerweise liegt die Schätzfunktion an der Ausgangsschicht vor. Der Ausgang der Analyseeinheit kann der Ausgangsschicht des künstlichen neuronalen Netzes entsprechen. Vorzugsweise ist das künstliche neuronale Netz dafür ausgebildet, in Abhängigkeit von den Startdaten und/oder von der ermittelten Substanzklasse und/oder von der ermittelten Schätzfunktion die gewichteten Verbindungen festzulegen, insbesondere wenn das künstliche neuronale Netz das rekurrente künstliche neuronale Netz ist. Das künstliche neuronale Netz kann typischerweise in Programmcodemitteln abgebildet und/oder in der Recheneinheit ausgeführt werden.

Eine Ausführungsform sieht vor, dass das künstliche neuronale Netz gemäß den Startdaten trainiert wird. Das Training kann beispielsweise derart erfolgen, dass beispielsweise eine Trainingsdatenbank mit einer Menge an Trainings-Substanzklassen und zugehörigen Trainings-Protokolldaten verwendet wird. Vorteilhafterweise kann das Training laufend erfolgen, insbesondere wenn das künstliche neuronale Netz lernend bzw. rekurrent ausgebildet ist. Vorzugsweise wird das künstliche neuronale Netz, insbesondere die gewichteten Verbindungen, gemäß den Startdaten angepasst. Das künstliche neuronale Netz kann in diesem Fall gemäß einem Entscheidungsbaum ausgebildet sein, welcher jeweils in Abhängigkeit von der bereitgestellten biologischen Testsubstanz angepasst wird. Das Training kann beispielsweise derart erfolgen, dass an der Eingangsschicht die Startdaten, insbesondere die zugrundeliegenden Protokolldaten, und an der Ausgangsschicht die zugehörige Menge an Substanzklassen anliegt, wodurch üblicherweise die gewichteten Verbindungen angepasst werden. Die gewichteten Verbindungen können vorzugsweise beim Ermitteln der Substanzklasse, insbesondere beim Ermitteln der Schätzfunktion, angepasst werden. Diese Ausführungsform bietet insbesondere einen Vorteil, dass das künstliche neuronale Netz in Abhängigkeit von den Startdaten angepasst wird. Dies ermöglicht vorzugsweise ein schnelleres Ermitteln der Substanzklasse des nosokomialen Keims. In diesem Fall sind die Startdaten typischerweise ein Gedächtnis des künstlichen neuronalen Netzes.

Eine Ausführungsform sieht vor, dass die Datenverarbeitungseinheit einen Bayes-Schätzer aufweist, welcher gemäß den Startdaten initialisiert wird, und wobei die Schätzfunktion in der Datenverarbeitungseinheit gemäß dem Bayes-Schätzer ermittelt wird. Vorteilhafterweise ermöglicht der Bayes-Schätzer eine besonders vorteilhafte Kombination von den Protokolldaten und den Ursprungsdaten. Grundsätzlich bietet der Bayes-Schätzer einen Vorteil einer im Hinblick auf eine Rechenleistung anspruchsloseren Anforderung, insbesondere im Vergleich zum künstlichen neuronalen Netz. Vorteilhafterweise kann der Bayes-Schätzer mit dem künstlichen neuronalen Netz derart kombiniert werden, dass die Datenverarbeitungseinheit leistungsfähiger und/oder präziser wird. Vorteilhafterweise ist eine Datenverarbeitungseinheit lediglich mit dem Bayes-Schätzer günstiger als die Datenverarbeitungseinheit mit dem künstlichen neuronalen Netz oder als die Datenverarbeitungseinheit mit dem künstlichen neuronalen Netz sowie mit dem Bayes-Schätzer.

In diesem Ausführungsbeispiel liegen die Startdaten üblicherweise als A-priori-Wahrscheinlichkeitsverteilung und/oder die Schätzfunktion als A-posteriori-Wahrscheinlichkeitsverteilung vor. Das Ermitteln der Schätzfunktion umfasst insbesondere ein Auswerten der A-posteriori-Wahrscheinlichkeitsverteilung. Der Bayes-Schätzer ermöglicht insbesondere ein Verwenden des Bayes'schen Theorems für das Ermitteln der Substanzklasse des nosokomialen Keims. Der Bayes-Schätzer kann typischerweise in Programmcodemitteln abgebildet und/oder in der Recheneinheit ausgeführt werden.

Eine Ausführungsform sieht vor, dass das Datenbanksystem ein weiteres künstliches neuronales Netz aufweist und wobei mittels des weiteren künstlichen neuronalen Netzes die Startdaten in Abhängigkeit von den Ursprungsdaten festgelegt und an die Analyseeinheit übertragen werden. Diese Ausführungsform bietet insbesondere einen Vorteil einer hochgradigen Automatisierung des Verfahrens für das Ermitteln der Substanzklasse des nosokomialen Keims. Vorteilhafterweise kann das weitere künstliche neuronale Netz, insbesondere aufgrund einer Anpassungsfähigkeit, die Startdaten besser festlegen, als wenn die Startdaten beispielsweise durch einen Nutzer und/oder gemäß starren Gewichten festgelegt werden.

Grundsätzlich kann in Hinblick auf eine Topologie das weitere künstliche neuronale Netz wie das künstliche neuronale Netz ausgebildet sein. Daher wird aus Gründen der Übersichtlichkeit hiermit auf die obige Beschreibung des künstlichen neuronalen Netzes verwiesen. Das weitere künstliche neuronale Netz kann insbesondere das Abstandsmaß, insbesondere für das Klassifizieren, und/oder eine bestimmte Wahrscheinlichkeit festlegen. Das weitere künstliche neuronale Netz wird beispielsweise unter Verwendung der Menge an Substanzklassen und den zugehörigen Protokolldaten trainiert. Beispielsweise kann das weitere künstliche neuronale Netz dadurch lernen, welche Substanzklasse insbesondere in Abhängigkeit von der Zeitinformation und/oder Ortinformation mit der bestimmten Wahrscheinlichkeit, insbesondere mit der A-priori-Wahrscheinlichkeitsverteilung, auftreten und/oder ermittelt werden kann, wobei die bestimmte Wahrscheinlichkeit insbesondere die Startdaten bilden kann. Das weitere künstliche neuronale Netz und das künstliche neuronale Netz können grundsätzlich als ein einzelnes künstliches neuronales Netz trainiert und/oder ausgebildet sein.

Eine Ausführungsform sieht vor, dass die Protokolldaten und/oder die Ursprungsdaten eine Zeitinformation und/oder Ortsinformation über das Vorkommen des zugeordneten zumindest einen nosokomialen Keims aufweisen. Mittels der Zeitinformation und/oder der Ortsinformation kann vorzugsweise berücksichtigt werden, inwiefern es wahrscheinlich ist, dass zu einem gewissen Zeitpunkt und/oder an einem bestimmten Ort die Substanzklasse des nosokomialen Keims ermittelt wird. In anderen Worten kann vorzugsweise in Abhängigkeit von der Zeitinformation und/oder Ortsinformation ausgeschlossen werden, dass anstatt der Substanzklasse des nosokomialen Keims eine weitere Substanzklasse ermittelt wird. Die Ortsinformation kann typischerweise in ein lokales oder regionales oder nationales Gebiet unterteilt werden.

Grundsätzlich ist es denkbar, dass die Protokolldaten alternativ oder zusätzlich eine Information über eine beim Bereitstellen der biologischen Testsubstanz verwendete Bereitstellungseinheit aufweisen. Die Bereitstellungseinheit kann ein medizinisches Gerät, beispielsweise ein Autoklav sein. Weiterhin ist es denkbar, dass die Protokolldaten eine Information über einen die biologische Testsubstanz bereitstellenden Nutzer aufweisen.

Eine Ausführungsform sieht vor, dass die Ortsinformation einen Untersuchungsraum, eine Abteilung in einem Krankenhaus, das Krankenhaus und/oder einen Ort außerhalb des Krankenhauses beschreibt. Der Untersuchungsraum und/oder die Abteilung in dem Krankenhaus sind typischerweise dem lokalen Gebiet zuzuordnen. Der Ort außerhalb des Krankenhaus ist typischerweise dem regionalen oder nationalen Gebiet zuzuordnen, wobei sich typischerweise das regionale oder nationale Gebiet in einer Distanz zum Krankenhaus unterscheiden lässt. Eine Größenordnung der Distanz im regionalen und/oder im nationalen Gebiet kann Kilometer sein. Die Distanz im regionalen Gebiet ist beispielsweise kleiner 50 km. Die Distanz im nationalen Gebiet ist beispielsweise größer oder gleich 50 km.

Diese Ausführungsform ist insofern vorteilhaft, weil ein Vorkommen von Keimen, insbesondere des nosokomialen Keims, stark von dem Ursprung der biologischen Testsubstanz abhängen kann. Diese Ausführungsform ermöglicht vorteilhafterweise ein Festlegen von unterschiedlichen Startdaten, welche basierend auf durchgeführten Tätigkeiten wie Operationen, Pflege, Therapie und/oder Diagnostik an von den Ortsinformationen beschriebenen Orten variieren können.

Eine Ausführungsform sieht vor, dass die Zeitinformation die Aufenthaltsdauer des Patienten und/oder einen Zeitpunkt des Ursprungs der biologischen Testsubstanz beschreibt. Vorteilhafterweise kann das Ermitteln der Substanzklasse schnell erfolgen, weil lediglich zeitlich relevante Substanzklassen gemäß den jeweils zugeordneten Protokolldaten beim Festlegen der Startdaten berücksichtigt werden.

Grundsätzlich ist es denkbar, dass die Protokolldaten insbesondere die Zeitinformation und die Ortsinformation aufweisen. Üblicherweise hängen die Zeitinformation und die Ortsinformation voneinander ab. In diesem Fall weisen die Startdaten und/oder die Schätzfunktion, insbesondere die A-priori-Wahrscheinlichkeitsverteilung und/oder die A-posteriori-Wahrscheinlichkeitsverteilung eine zeitliche Dimension und/oder eine räumliche Dimension auf.

Eine Ausführungsform sieht vor, dass nach dem Ermitteln der Substanzklasse die ermittelte Substanzklasse des nosokomialen Keims in das Datenbanksystem übertragen wird und wobei zusätzlich zur ermittelten Substanzklasse des nosokomialen Keims die Ursprungsdaten in das Datenbanksystem übertragen und als Protokolldaten gespeichert werden. Die Ausführungsform bietet insbesondere einen Vorteil, dass das Datenbanksystem mit keimspezifischen Ursprungsdaten aktualisiert werden kann. Vorteilhafterweise kann das Datenbanksystem gemäß den Ursprungsdaten ausgebildet werden. Weiterhin kann vorteilhaft sein, insbesondere wenn die Protokolldaten die Zeitinformation und/oder die Ortsinformation aufweisen, dass das Datenbanksystem Krankenhaus- und/oder ortsspezifische und/oder historische Daten enthält, wodurch das Ermitteln der Substanzklasse verbessert wird.

Die ermittelte Substanzklasse und/oder die Ursprungsdaten werden typischerweise von der Analyseeinheit, insbesondere von der Recheneinheit der Analyseeinheit, an das Datenbanksystem übertragen. Das Datenbanksystem kann in den der Substanzklasse zugehörigen Protokolldaten üblicherweise einen Zeitpunkt des Übertragens der ermittelten Substanzklasse unabhängig davon, ob Ursprungsdaten übertragen werden, ergänzen. Wenn die Ursprungsdaten übertragen werden, entstehen üblicherweise aus den Protokolldaten weitere Protokolldaten, wobei die weiteren Protokolldaten die Ursprungsdaten aufweisen. Die Ursprungsdaten der biologischen Testsubstanz werden typischerweise der ermittelten Substanzklasse in dem Datenbanksystem hinzugefügt. Das Datenbanksystem kann insbesondere die ermittelte Substanzklasse aus der Menge an Substanzklassen bestimmen, um beispielsweise die Protokolldaten der aus der Menge an Substanzklassen bestimmten Substanzklassen für ein erneutes Festlegen der Startdaten zu verwenden und/oder um die Ursprungsdaten zu ergänzen. Wenn beispielsweise die Menge an Substanzklassen die ermittelte Substanzklasse nicht enthält, kann das Datenbanksystem beispielsweise die Menge an Substanzklassen um die ermittelte Substanzklasse erweitern, wobei die Ursprungsdaten als Protokolldaten der ermittelten Substanzklasse im Datenbanksystem gespeichert werden.

Eine Ausführungsform sieht vor, dass die Ursprungsdaten des ermittelten nosokomialen Keims mit den Protokolldaten der zumindest einen Substanzklasse aus der Menge an Substanzklassen verglichen werden, womit eine Abschätzung über eine Verbreitung der ermittelten Substanzklasse des nosokomialen Keims erstellt wird und wobei in Abhängigkeit von der Abschätzung und von einem Schwellwert eine Ausgabeinformation über die Verbreitung der ermittelten Substanzklasse bereitgestellt wird. Die Ausführungsform ist vorteilhaft, weil vorzugsweise die Protokolldaten und/oder die Ursprungsdaten Ortsinformationen umfassen, welche sich in dem lokalen oder regionalen oder nationalen Gebiet unterscheiden. Vorteilhafterweise beschreibt also die Ausgabeinformation, inwiefern der ermittelte Keim massenhaft und/oder epidemisch, insbesondere in dem lokalen oder regionalen oder nationalen Gebiet, auftritt.

Das Vergleichen erfolgt typischerweise im Datenbanksystem, in der Recheneinheit und/oder in der Analyseeinheit. Die Abschätzung kann in Abhängigkeit von der ermittelten Substanzklasse, insbesondere von der dritten Kategorie der Substanzklasse mit dem Stammbaum, erfolgen. Beispielsweise kann dadurch eine spezifischer nosokomialer Keim und/oder eine Gattung mit mehreren nosokomialen Keimen berücksichtigt werden. Die Abschätzung weist insbesondere eine relative Häufigkeit des ermittelten Keims typischerweise mit einer zeitlichen Dimension und/oder einer räumlichen Dimension auf. Der Schwellwert kann eine Ermittlungsgrenzrate beschreiben, wobei die Ermittlungsgrenzrate eine Häufigkeit des ermittelten Keims pro Zeiteinheit aufweist. Die Ermittlungsgrenzrate kann alternativ oder zusätzlich zu der zeitlichen Dimension eine örtliche Dimension aufweisen, wobei die örtliche Dimension in Maßeinheiten und/oder in Räumen des Krankenhauses gemessen wird. Die Ausgabeinformation kann vorzugsweise automatisch innerhalb des Datenbanksystems, insbesondere wenn das Datenbanksystem die multizentrische Datenbank aufweist, übertragen werden und/oder einen Alarm, insbesondere in zumindest einem der mehreren Krankenhäusern der multizentrischen Datenbank, auslösen.

Eine Ausführungsform sieht vor, dass nach der Behandlung des Patienten gemäß der ermittelten Substanzklasse des nosokomialen Keims ein Gesundheitszustand des Patienten und/oder eine Behandlungsmethode des Patienten in das Datenbanksystem übertragen und der ermittelten Substanzklasse des nosokomialen Keims zugeordnet wird. Vorteilhafterweise kann dadurch das Datenbanksystem um ein Resistenzspektrum der Substanzklasse, insbesondere des ermittelten nosokomialen Keims, ergänzt werden, insbesondere wenn alternativ oder zusätzlich zum Gesundheitszustand die, insbesondere bevorzugte und/oder unvorteilhafte, Behandlungsmethode des ermittelten nosokomialen Keims in das Datenbanksystem übertragen wird.

Der Gesundheitszustand des Patienten und/oder die Behandlungsmethode des nosokomialen Keims können beispielsweise als der ermittelten Substanzklasse zugehörige Protokolldaten abgespeichert werden. Beispielsweise kann das Resistenzspektrum derart ermittelt werden, dass eine medikamentöse Behandlungsmethode des ermittelten nosokomialen Keims in Abhängigkeit von dem Gesundheitszustand des Patienten nach der Behandlung bewertet wird. Vorteilhafterweise werden bevorzugte Behandlungsmethoden und/oder unvorteilhafte Behandlungsmethoden in dem Datenbanksystem abgespeichert. Die bevorzugte Behandlungsmethode des ermittelten nosokomialen Keims führt vorzugsweise zu einem positiveren Gesundheitszustand des Patienten als die unvorteilhafte Behandlungsmethode.

Ein medizinisches System weist das Datenbanksystem und die Analyseeinheit auf.

Ein Computerprogrammprodukt, welches direkt in einen Speicher der Recheneinheit, insbesondere des medizinischen Systems, ladbar ist, weist Programmcodemittel auf, um das Verfahren für das Ermitteln der Substanzklasse des nosokomialen Keims in der biologischen Testsubstanz auszuführen, wenn das Computerprogrammprodukt in der Recheneinheit ausgeführt wird.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Verfahren für ein Ermitteln einer Substanzklasse eines nosokomialen Keims in einer biologischen Testsubstanz eines Patienten in einem ersten Ausführungsbeispiel,
Fig. 2 das Verfahren in einem zweitem Ausführungsbeispiel und
Fig. 3 ein medizinisches System.

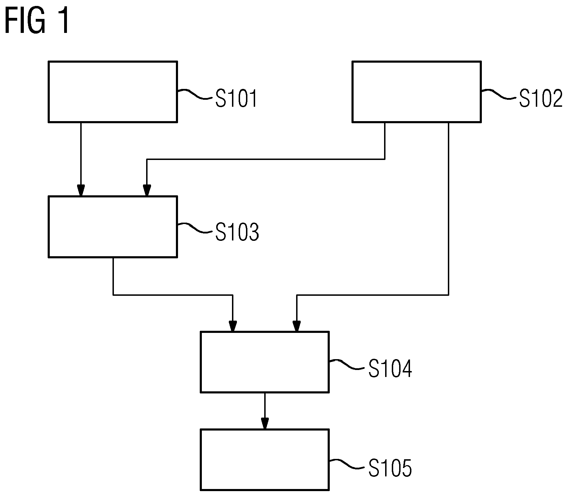

**Fig. 1** zeigt ein Flussdiagramm eines ersten Ausführungsbeispiels des Verfahrens für ein Ermitteln einer Substanzklasse eines nosokomialen Keims in einer biologischen Testsubstanz eines Patienten.

Verfahrensschritt S101 kennzeichnet ein Bereitstellen eines Datenbanksystems, welches eine Menge an Substanzklassen aufweist, wobei jeder Substanzklasse zumindest ein nosokomialer Keim zugeordnet ist und zumindest eine Substanzklasse aus der Menge an Substanzklassen Protokolldaten über ein Vorkommen des zugeordneten zumindest einen nosokomialen Keims aufweist.

Verfahrensschritt S102 kennzeichnet ein Bereitstellen der biologischen Testsubstanz des Patienten zusammen mit Ursprungsdaten, welche einen Ursprung der biologischen Testsubstanz beschreiben.

Verfahrensschritt S103 kennzeichnet ein Festlegen von Startdaten durch ein Gewichten der Protokolldaten aus der Menge an Substanzklassen in Abhängigkeit von den Ursprungsdaten. Verfahrensschritt S104 kennzeichnet ein Ermitteln einer Schätzfunktion mittels einer Analyseeinheit, wobei die Startdaten und die biologische Testsubstanz als Eingangsparameter in die Analyseeinheit eingehen und die Schätzfunktion an einem Ausgang der Analyseeinheit bereitgestellt wird.

Verfahrensschritt S105 kennzeichnet ein Ermitteln der Substanzklasse des nosokomialen Keims in der biologischen Testsubstanz des Patienten gemäß der Schätzfunktion.

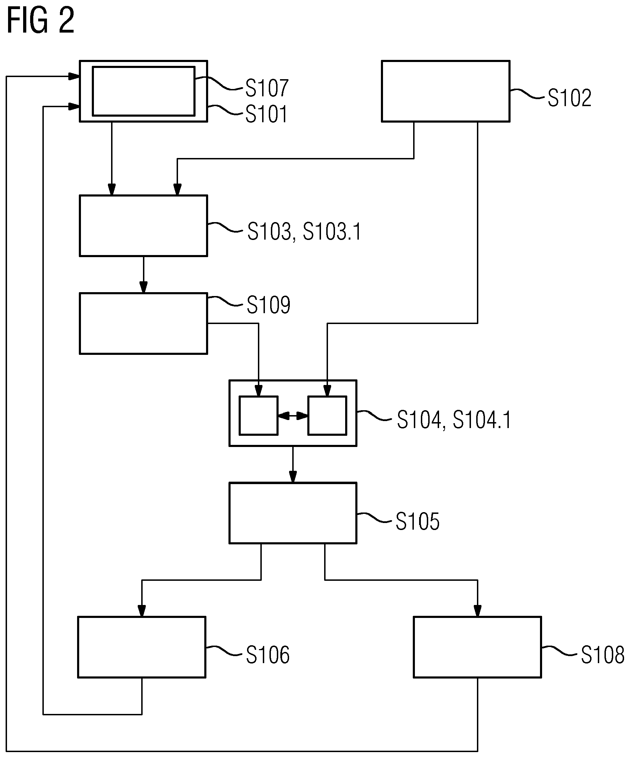

**Fig. 2** zeigt ein Flussdiagramm eines zweiten Ausführungsbeispiels des Verfahrens für ein Ermitteln einer Substanzklasse eines nosokomialen Keims in einer biologischen Testsubstanz eines Patienten.

Verfahrensschritt S106 kennzeichnet, dass nach dem Ermitteln der Substanzklasse die ermittelte Substanzklasse des nosokomialen Keims in das Datenbanksystem übertragen wird und wobei zusätzlich zur ermittelten Substanzklasse des nosokomialen Keims die Ursprungsdaten in das Datenbanksystem übertragen und als Protokolldaten gespeichert werden.

Verfahrensschritt S107 kennzeichnet, dass die Ursprungsdaten des ermittelten nosokomialen Keims mit den Protokolldaten der zumindest einen Substanzklasse aus der Menge an Substanzklassen verglichen werden, womit eine Abschätzung über eine Verbreitung der ermittelten Substanzklasse des nosokomialen Keims erstellt wird und wobei in Abhängigkeit von der Abschätzung und von einem Schwellwert eine Ausgabeinformation über die Verbreitung der ermittelten Substanzklasse bereitgestellt wird.

Verfahrensschritt S108 kennzeichnet, dass nach der Behandlung des Patienten gemäß der ermittelten Substanzklasse des nosokomialen Keims ein Gesundheitszustand des Patienten und/oder eine Behandlungsmethode des Patienten in das Datenbanksystem übertragen und der ermittelten Substanzklasse des nosokomialen Keims zugeordnet wird.

Fig. 2 zeigt in Verfahrensschritt S104.1, dass die Analyseeinheit eine biologische Testsubstanzauswerteeinheit und eine Datenverarbeitungseinheit aufweist und wobei für das Ermitteln der Schätzfunktion die biologische Testsubstanz als Eingangsparameter in die biologische Testsubstanzauswerteeinheit eingehen und die Startdaten als Eingangsparameter in die Datenverarbeitungseinheit eingehen. Weiterhin ist in S104.1 gezeigt, dass für das Ermitteln der Schätzfunktion vorläufige Ergebnisse der Schätzfunktion zwischen der biologischen Testsubstanzauswerteeinheit und der Datenverarbeitungseinheit ausgetauscht werden.

Verfahrensschritt S109 kennzeichnet, dass für das Ermitteln der Schätzfunktion die Datenverarbeitungseinheit ein künstliches neuronales Netz aufweist und das künstliche neuronale Netz gemäß den Startdaten trainiert wird.

Verfahrensschritt S109 kann kennzeichnen, dass alternativ oder zusätzlich die Datenverarbeitungseinheit einen Bayes-Schätzer aufweist, welcher gemäß den Startdaten initialisiert wird, und wobei die Schätzfunktion in der Datenverarbeitungseinheit gemäß dem Bayes-Schätzer ermittelt wird.

Verfahrensschritt S109 kann also insbesondere das Trainieren und/oder das Initialisieren der Datenverarbeitungseinheit kennzeichnen, wobei die Datenverarbeitungseinheit insbesondere gemäß Verfahrensschritten S104, S104.1 verwendet werden kann.

Verfahrensschritt S103.1 kennzeichnet, dass das Datenbanksystem ein weiteres künstliches neuronales Netz aufweist und wobei mittels des weiteren künstlichen neuronalen Netzes die Startdaten in Abhängigkeit von den Ursprungsdaten festgelegt und an die Analyseeinheit übertragen werden.

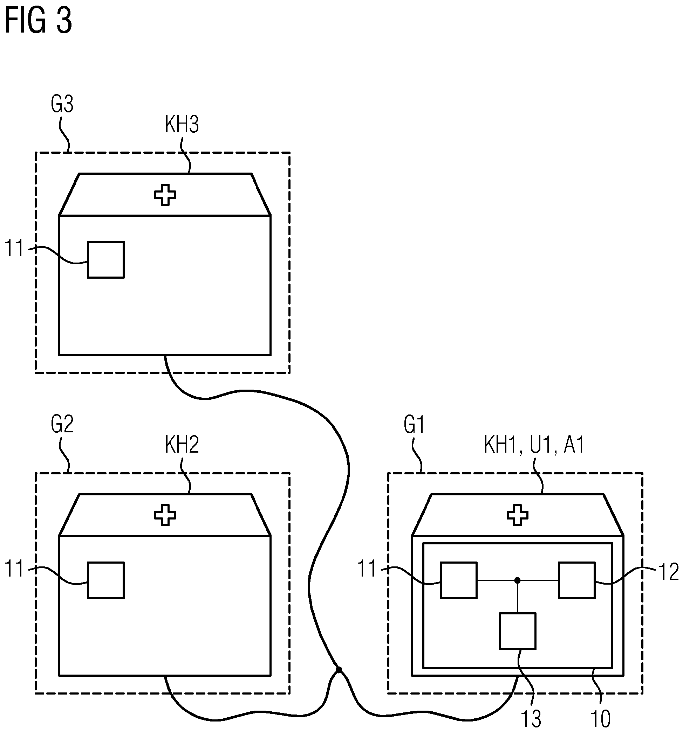

**Fig. 3** zeigt ein medizinisches System 10, welches ein Datenbanksystem 11 und die Analyseeinheit 12 aufweist. Das medizinische System 10 weist in diesem Ausführungsbeispiel zusätzlich eine Recheneinheit 13 auf. Die Recheneinheit 13 ist mit dem Datenbanksystem 11 und der Analyseeinheit zum Austausch der Protokolldaten, der Ursprungsdaten und/oder der ermittelten Substanzklasse verbunden.

In diesem Ausführungsbeispiel weisen die Protokolldaten und/oder die Ursprungsdaten eine Zeitinformation und/oder Ortsinformation über das Vorkommen des zugeordneten zumindest einen nosokomialen Keims auf, wobei die Ortsinformation einen Untersuchungsraum U1, eine Abteilung A1 in einem Krankenhaus KH1, das Krankenhaus KH1 und/oder einen Ort KH2, KH3 außerhalb des Krankenhauses beschreibt und wobei die Zeitinformation eine Aufenthaltsdauer des Patienten und/oder einen Zeitpunkt des Ursprungs der biologischen Testsubstanz beschreibt. Das medizinische System 10 kann beispielsweise innerhalb des Krankenhaus KH1, insbesondere zwischen Untersuchungsraum U1 und Abteilung A1, und/oder Krankenhausübergreifend verteilt angeordnet sein.

Fig. 3 zeigt, dass ein lokales Gebiet G1 das Krankenhaus KH1 aufweist, dass ein regionales Gebiet G2 das regionale Krankenhaus KH2 aufweist und dass ein nationales Gebiet G3 das weitere Krankenhaus KH3 aufweist. Das regionale Gebiet G2 ist vom Krankenhaus KH1 beispielsweise weniger als 50 km entfernt. Das nationale Gebiet G3 ist vom Krankenhaus KH1 beispielweise mindestens 50 km entfernt. Das Datenbanksystem 11 ist eine multizentrische Datenbank, welche über das Krankenhaus KH1, das regionale Krankenhaus KH2 und das nationale Krankenhaus KH3 verteilt angeordnet und/oder üblicherweise mittels eines Internets und/oder eines Netzwerks verbunden ist.

Beispielsweise kann gemäß den Verfahrensschritten S106 und S107 die Ausgabeinformation im regionalen Krankenhaus KH2 und/oder im weiteren Krankenhaus KH3 bereitgestellt werden, wenn im Krankenhaus KH1 die biologische Testsubstanz bereitgestellt wird und/oder die Substanzklasse des nosokomialen Keims in der biologischen Testsubstanz ermittelt wird. In anderen Worten wird die ermittelte Substanzklasse des nosokomialen Keim in der biologischen Testsubstanz des Patienten vorzugsweise in die über die mehreren Krankenhäuser KH1, KH2, KH3 verteilte multizentrische Datenbank übertragen, um insbesondere weitere Patienten vor dem nosokomialen Keim bewahren zu können.

Beispielsweise können die Gewichte beim Gewichten der Protokolldaten aus der Menge an Substanzklassen in Abhängigkeit von den Ursprungsdaten gemäß einer Distanz zwischen den Ortsinformationen der Protokolldaten festgelegt werden. Die Distanz zwischen den Ortsinformationen der Protokolldaten kann beispielsweise von dem lokalen Gebiet G1, dem regionalen Gebiet G2 und/oder dem nationalen Gebiet G3 abhängen.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Ermitteln einer Substanzklasse eines nosokomialen Keims in einer biologischen Testsubstanz eines Patienten mit den folgenden Schritten:
- Bereitstellen eines Datenbanksystems, welches eine Menge an Substanzklassen aufweist, wobei jeder Substanzklasse zumindest ein nosokomialer Keim zugeordnet ist und zumindest eine Substanzklasse aus der Menge an Substanzklassen Protokolldaten über ein Vorkommen des zugeordneten zumindest einen nosokomialen Keims aufweist,
- Bereitstellen der biologischen Testsubstanz des Patienten zusammen mit Ursprungsdaten, welche einen Ursprung der biologischen Testsubstanz beschreiben,
- Festlegen von Startdaten durch ein Gewichten der Protokolldaten aus der Menge an Substanzklassen in Abhängigkeit von den Ursprungsdaten,
- Ermitteln einer Schätzfunktion mittels einer Analyseeinheit, wobei die Startdaten und die biologische Testsubstanz als Eingangsparameter in die Analyseeinheit eingehen und die Schätzfunktion an einem Ausgang der Analyseeinheit bereitgestellt wird, und
- Ermitteln der Substanzklasse des nosokomialen Keims in der biologischen Testsubstanz des Patienten gemäß der Schätzfunktion.

2. Verfahren nach Anspruch 1, wobei die Analyseeinheit eine biologische Testsubstanzauswerteeinheit und eine Datenverarbeitungseinheit aufweist und wobei für das Ermitteln der Schätzfunktion die biologische Testsubstanz als Eingangsparameter in die biologische Testsubstanzauswerteeinheit eingehen und die Startdaten als Eingangsparameter in die Datenverarbeitungseinheit eingehen.

3. Verfahren nach Anspruch 2, wobei für das Ermitteln der Schätzfunktion vorläufige Ergebnisse der Schätzfunktion zwischen der biologischen Testsubstanzauswerteeinheit und der Datenverarbeitungseinheit ausgetauscht werden.

4. Verfahren nach einem der Ansprüche 2 bis 3, wobei für das Ermitteln der Schätzfunktion die Datenverarbeitungseinheit ein künstliches neuronales Netz aufweist.

5. Verfahren nach Anspruch 4, wobei das künstliche neuronale Netz gemäß den Startdaten trainiert wird.

6. Verfahren nach einem der Ansprüche 2 bis 5, wobei die Datenverarbeitungseinheit einen Bayes-Schätzer aufweist, welcher gemäß den Startdaten initialisiert wird, und wobei die Schätzfunktion in der Datenverarbeitungseinheit gemäß dem Bayes-Schätzer ermittelt wird.

7. Verfahren nach einem der vorhergehenden Ansprüche, wobei das Datenbanksystem ein weiteres künstliches neuronales Netz aufweist und wobei mittels des weiteren künstlichen neuronalen Netzes die Startdaten in Abhängigkeit von den Ursprungsdaten festgelegt und an die Analyseeinheit übertragen werden.

8. Verfahren nach einem der vorhergehenden Ansprüche, wobei die Protokolldaten und/oder die Ursprungsdaten eine Zeitinformation und/oder Ortsinformation über das Vorkommen des zugeordneten zumindest einen nosokomialen Keims aufweisen.

9. Verfahren nach Anspruch 8, wobei die Ortsinformation einen Untersuchungsraum, eine Abteilung in einem Krankenhaus, das Krankenhaus und/oder einen Ort außerhalb des Krankenhauses beschreibt.

10. Verfahren nach einem der Ansprüche 8 bis 9, wobei die Zeitinformation eine Aufenthaltsdauer des Patienten und/oder einen Zeitpunkt des Ursprungs der biologischen Testsubstanz beschreibt.

11. Verfahren nach einem der vorhergehenden Ansprüche, wobei nach dem Ermitteln der Substanzklasse die ermittelte Substanzklasse des nosokomialen Keims in das Datenbanksystem übertragen wird und wobei zusätzlich zur ermittelten Substanzklasse des nosokomialen Keims die Ursprungsdaten in das Datenbanksystem übertragen und als Protokolldaten gespeichert werden.

12. Verfahren nach Anspruch 11, wobei die Ursprungsdaten des ermittelten nosokomialen Keims mit den Protokolldaten der zumindest einen Substanzklasse aus der Menge an Substanzklassen verglichen werden, womit eine Abschätzung über eine Verbreitung der ermittelten Substanzklasse des nosokomialen Keims erstellt wird und wobei in Abhängigkeit von der Abschätzung und von einem Schwellwert eine Ausgabeinformation über die Verbreitung der ermittelten Substanzklasse bereitgestellt wird.

13. Verfahren nach einem der vorhergehenden Ansprüche, wobei nach der Behandlung des Patienten gemäß der ermittelten Substanzklasse des nosokomialen Keims ein Gesundheitszustand des Patienten und/oder eine Behandlungsmethode des Patienten in das Datenbanksystem übertragen und der ermittelten Substanzklasse des nosokomialen Keims zugeordnet wird.

14. Medizinisches System, aufweisend das Datenbanksystem und die Analyseeinheit, wobei das medizinische System zu einem Verfahren nach einem der vorhergehenden Ansprüchen ausgebildet ist.

15. Computerprogrammprodukt, welches direkt in einen Speicher einer Recheneinheit ladbar ist, mit Programmcodemitteln, um ein Verfahren nach einem der Ansprüche 1 bis 13 auszuführen, wenn das Computerprogrammprodukt in der Recheneinheit ausgeführt wird.
